# EUROPEAN PATENT APPLICATION

(11) **EP 4 245 745 A1**
(43) Date of publication of application: **20.09.2023**
(21) Application number: 21891789.6
(22) Date of filing: 05.11.2021
(51) Int. Cl.: C07C 29/76, C07C 29/86, C07C 35/02, A24B 15/167, A24B 15/26, A24B 13/00

(54) **TOBACCO EXTRACT CONTAINING CEMBRATRIENDIOL AND PRODUCTION METHOD THEREFOR**

(30) Priority: 13.11.2020 JP 2020189330
(71) Applicant: Japan Tobacco Inc., Tokyo 105-6927 (JP)
(72) Inventor: CHIDA, Masahiro, Tokyo 130-8603 (JP); MIZUTANI, Masashi, Tokyo 130-8603 (JP); MATSUMOTO, Yuichi, Tokyo 130-8603 (JP); TSURUOKA, Naoya, Tokyo 130-8603 (JP)
(74) Representative: Hoffmann Eitle
(86) International application number: PCT/JP2021/040834
(87) International publication number: WO 2022/102543

(57) **Abstract**

Provided is a method for producing a tobacco extract containing cembratrienediol, the method including: a process 1 that includes preparing a discharged solid obtained by an expansion treatment of a tobacco raw material; a process 2 that includes subjecting the discharged solid to extraction with an aprotic solvent and water; and a process 3 that includes recovering an organic phase from the process 2.

## Description

### TECHNICAL FIELD

The present invention relates to a tobacco extract containing cembratrienediol and a method for producing the same.

### BACKGROUND ART

Cembranoids are secondary metabolites secreted from glandular hairs in tobacco plant epidermis, and are components exhibiting various physiological activities of biological control. The main component of cembranoid is cembratrienediol (CBT), which is correlated with the characteristic aroma of tobacco plants, and thus cembratrienediol has been focused as a component important for expression of aroma quality. The content of the cembratrienediol component is the largest among so-called 89 types of cembranoid compounds reported in other tobacco plant leaf surface resin components, and cembratrienediol is the most secreted component among the cembranoid compounds (NPL 1). CBT is a monocyclic diterpenoid found in leachate of trichome. There are isomers of CTB, and α-CBT and β-CBT are known. These CBTs have attracted attention as an anticarcinogenic agent, as with other cembranoids (PTLs 1 and 2, NPL 2). For example, cembratrienediol (CBT) isolated from tobacco leaves has attracted attention as a potent antitumor agent (PTL 5).

In general, CBT is known to be obtained as follows. For example, leaf surface resin components are extracted from raw leaves of tobacco plants or dried tobacco leaves through immersion extraction with an organic solvent such as hexane, ether, chloroform, dichloromethane, methanol, or ethanol. The leaf surface resin components are concentrated, and then produced through isolation by, for example, chromatography using silica gel (PTLs 3 and 4, and NPL 3). In addition, various extraction methods have been reported (NPL 4 and PTL 5). However, considering the expansion of effective use of CBT, the total amount of purified products obtained from tobacco leaves is very small. For example, PTL 5 states that 6 g of α-CBT and 2 g of β-CBT were obtained from 10 kg of dried tobacco leaves.

In addition, many methods using liquefied carbon dioxide gas or supercritical carbon dioxide gas and taking advantage of the characteristics of the target components have been studied (PTLs 6 and 7). On the other hand, raw materials expanded with liquefied carbon dioxide gas have been studied as one of the raw materials for cigarettes, and the process is very similar to the extraction process using carbon dioxide gas described above. Therefore, a method has been studied in which a part of the expansion process is applied (PTL 8). PTL 8 discloses a method for obtaining tobacco aroma components such as CBT, in which carbon dioxide in a supercritical state is brought into contact with a tobacco raw material to dissolve tobacco components in the carbon dioxide, and fat-soluble ingredients are separated from the tobacco components dissolved in the carbon dioxide, and recovered. In these methods, components dissolved in vaporized carbon dioxide are focused, and it has been necessary to separately provide a gas-liquid separator for separating gas and tobacco components.

### CITATION LIST

### PATENT LITERATURE

PTL 1: US Patent No. 7977384
PTL 2: US Patent Application Publication No. 2005/025075
PTL 3: US Patent No. 4701570
PTL 4: US Patent Application Publication No. 2013/0014771
PTL 5: CN Patent Application Publication No. 104000302 A
PTL 6: International Publication No. WO 2007/029264
PTL 7: GB Patent No. 2173985
PTL 8: International Publication No. WO 2007/119790

### NON PATENT LITERATURE

NPL 1: Yan, N.; Du, Y.; Liu, X.; Zhang, H.; Liu, Y.; Zhang, P.; Gong, D.; Zhang, Z. Chemical structures, biosynthesis, bioactivities, biocatalysis and semisynthesis of tobacco cembranoids: An overview. Ind. Crop. Prod. 2016, 83, pp66-80.
NPL 2: Hisao Takayanagi, Synthetic Study of Sarcophytol A, an Anticarcinogenic Cembranoid, 1995, Vol. 53, No. 8, p. 724 to 736
NPL 3: Y. Saito et. al., Identification of cembratriene-4,6-diol as antitumor-promoting agent from cigarette smoke condensate. Carcinogenesis 6:1189-1194 (1985)
NPL 4: I. Weini,et. al.. Application of response surface method to optimize purification of tobacco stem extract by molecular distillation[J]. Tobacco Science & Technology, 2019, 52(2):79-87.

### SUMMARY OF INVENTION

### TECHNICAL PROBLEM

In the conventional method, it has not been easy to efficiently obtain an extract containing cembratrienediol (CBT) from a tobacco raw material by a simple method. Therefore, an object of the present invention is to provide a method for efficiently producing an extract containing CBT from a discharged solid obtained by an expansion treatment of a tobacco raw material by a simple method.

### SOLUTION TO PROBLEM

The inventors have found that the above problem can be solved by using, as a raw material, a waste including tobacco stem or a solid extract discharged simultaneously with separation of carbon dioxide gas in the expansion treatment of a tobacco raw material. That is, the above problem is solved by the following present invention.
(1) A method for producing a tobacco extract containing cembratrienediol, the method including:
   a process 1 that includes preparing a discharged solid obtained by an expansion treatment of a tobacco raw material;
   a process 2 that includes subjecting the discharged solid to solid-liquid extraction with an aprotic solvent; and
   a process 3 that includes recovering an organic phase from the process 2.
(2) The production method according to (1), in which the process 2 further includes subjecting an aprotic solvent phase obtained by the solid-liquid extraction to extraction with water or an acid aqueous solution.
(3) The production method according to (1) or (2), in which the aprotic solvent is a water-insoluble organic solvent.
(4) The production method according to (3), in which the water-insoluble organic solvent is a hydrocarbon having 5 or 6 carbon atoms.
(5) The production method according to any one of (1) to (4), further including:
   a process P1 that includes subjecting an organic phase obtained in the process 2 or a tobacco extract obtained by removing the solvent from the organic phase to normal phase chromatography using a mixed solvent of n-hexane and ethyl acetate as a mobile phase, to obtain a fraction of n-hexane and ethyl acetate (50 : 50) and a fraction of n-hexane and ethyl acetate (40 : 60).
(6) The production method according to any one of (1) to (4), further including:
   a process P2 that includes adding methanol or ethanol to an organic phase obtained in the process 2 or a tobacco extract obtained by removing the solvent from the organic phase to produce a precipitate, and obtaining a solution from which the precipitate has been removed.
(7) The production method according to (5) or (6), further including:
   a process P3 that includes subjecting the fraction obtained in the process P1 or P2 to reverse phase chromatography using water and methanol as a mobile phase.
(8) The production method according to any one of (2) to (7), in which the acid aqueous solution is an aqueous solution of sulfuric acid, citric acid, or oxalic acid.
(9) The production method according to any one of (2) to (8), in which a pH of the acidic aqueous solution is 3 or less.
(10) A tobacco extract containing cembratrienediol produced by the method according to any one of (1) to (9).
(11) The tobacco extract according to (10), in which the tobacco extract contains 90 wt% or more of the cembratrienediol.
(12) A tobacco flavoring agent containing the tobacco extract according to (10) or (11).
(13) A tobacco material containing the tobacco flavoring agent according to (12).
(14) The tobacco material according to (13), in which the tobacco material is a tobacco sheet or a shredded tobacco.
(15) A tobacco rod part containing the tobacco material according to (13) or (14).
(16) A tobacco flavor inhalation article including the tobacco rod part according to (15).
(17) A non-combustion heating type tobacco flavor inhalation article or a non-combustion non-heating type tobacco flavor inhalation article including the tobacco rod part according to (15).
(18) A smokeless tobacco containing the tobacco material according to (13) or (14).

### ADVANTAGEOUS EFFECTS OF INVENTION

According to the present invention, it is possible to provide a method for efficiently producing a tobacco extract containing CBT by a simple method.

### BRIEF DESCRIPTION OF DRAWINGS

FIG. 1 is a GC/MS total ion chromatogram of an n-hexane solution obtained in Example 2.
FIG. 2 is a GC/MS total ion chromatogram of a chloroform solution obtained in Example 2.
FIG. 3 is a GC/MS total ion chromatogram of an ethyl acetate solution obtained in Example 2.
FIG. 4 is a GC/MS total ion chromatogram of an acetone solution obtained in Comparative Example 2.
FIG. 5 is a GC/MS total ion chromatogram of a methanol solution obtained in Comparative Example 2.
FIG. 6 is a GC/MS total ion chromatogram of an n-hexane solution obtained in Example 3.
FIG. 7 is a GC/MS total ion chromatogram of an ethyl acetate solution (concentration: 4 wt%) of a dried solid obtained from an eluate of n-hexane and ethyl acetate (50 : 50) fractionated by column chromatography in Example 4.
FIG. 8 is a GC/MS total ion chromatogram of an ethyl acetate solution (concentration: 4 wt%) of a dried solid obtained from an eluate of n-hexane and ethyl acetate (40 : 60) fractionated by column chromatography in Example 4.
FIG. 9 is a GC/MS total ion chromatogram of an ethyl acetate solution (concentration: 4 wt%) of a dried solid obtained from an eluate fractionated with an n-hexane single solvent by column chromatography in Example 4.
FIG. 10 is a chromatogram obtained by fractionating α-CBT and β-CBT by preparative high-speed liquid column chromatography in Example 5.
FIG. 11 is a view illustrating one embodiment of a non-combustion heating type tobacco flavor inhalation article.
FIG. 12 is a view illustrating one embodiment of a non-combustion heating type tobacco flavor inhalation system.
FIG. 13 is a view illustrating one embodiment of a non-combustion non-heating type tobacco flavor inhalation article.
FIG. 14 is a view illustrating one embodiment of a tobacco capsule.
FIG. 15 is a view illustrating one embodiment of a power supply unit.
FIG. 16 is a cross-sectional view of one embodiment of a cartridge.
FIG. 17 is a view illustrating an internal structure of one embodiment of the cartridge.
FIG. 18 is a view illustrating an outline of an expansion treatment process.

### DESCRIPTION OF EMBODIMENTS

Hereinafter, the present invention will be described in detail. In the present invention, the expression "X to Y" includes the end values X and Y.

### 1. Production method

The production method of the present invention includes: a process 1 that includes preparing a discharged solid obtained in an expansion treatment process of a tobacco raw material; a process 2 that includes subjecting the discharged solid to extraction with an aprotic solvent; and a process 3 that includes recovering an organic phase from the process 2.

### (1) Process 1

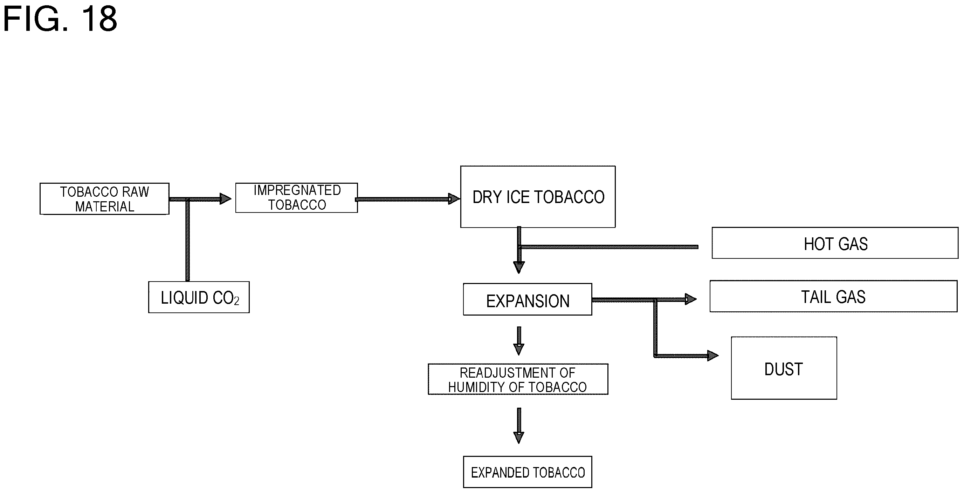

In this process, a discharged solid obtained in an expansion treatment process of a tobacco raw material is prepared. The expansion treatment refers to a treatment of impregnating a tobacco raw material with a liquid and rapidly vaporizing the liquid to increase the bulk of the tobacco material (see FIG. 18). In the present invention, a discharged solid discharged by a known expansion treatment can be used. Examples of the tobacco raw material include tobacco leaves, aged tobacco leaves, shredded tobacco, and tobacco powder. The tobacco leaves are a generic term for harvested tobacco leaves that have not undergone aging. One embodiment of aging includes curing. The shredded tobacco is obtained by shredding aged tobacco leaves or the like into a predetermined size. In the present invention, it is preferable to use, as the discharged solid, solid fat-soluble ingredients obtained when an expansion treatment is performed with supercritical carbon dioxide and carbon dioxide is separated from supercritical carbon dioxide containing tobacco components, as described in PTL 8. This is because the discharged solid obtained from the expansion treatment using supercritical carbon dioxide contains a large amount of CBT. In addition, since the expansion treatment process includes a process of threshing and shredding tobacco leaves as a raw material at the initial stage, tobacco fine powder and stems as a small amount of waste shreds are mixed into the tobacco raw material. When the raw material is subjected to an expansion treatment, after the expansion, light expanded shreds, heavy stems, and non-expanded shreds are discharged together with carbon dioxide gas, and become discharged solids. The discharged solids are the above-described solid fat-soluble ingredients, or solids of expanded shreds, stems, and non-expanded shreds, or mixtures thereof accordingly.

### (2) Process 2

In this process, the discharged solid is subjected to solid-liquid extraction with an aprotic solvent. The aprotic solvent is a solvent that is insoluble in water because it does not have a proton to be dissociated. Examples of the aprotic solvent include hydrocarbons; halogenated hydrocarbons such as dichloromethane and chloroform; esters such as ethyl acetate and propylene carbonate; ketones such as acetone; and nitriles such as acetonitrile. Among them, those having a boiling point of 70°C or lower are preferable from the viewpoint of being easily removed in a subsequent process. Specifically, hydrocarbons having 5 or 6 carbon atoms (heptane, hexane, etc.), which are water-insoluble organic solvents, are more preferable. The target CBT is transferred to the aprotic solvent (organic phase). The aprotic solvent can be classified into low-polar solvents and medium-polar solvents depending on their polarity. In one embodiment, based on the octanol/water partition coefficient (Kow), the aprotic medium-polar solvent is defined as a solvent exhibiting a positive value of log Kow of 2 or less, and the aprotic low-polar solvent is defined as a solvent exhibiting a value of log Kow of greater than 2 and 4 or less. In the present invention, the aprotic low-polar solvent is preferable. The hydrocarbon having 5 or 6 carbon atoms is the aprotic low-polar solvent. Kow is defined as the ratio of the concentration of the target compound (solvent) dissolved in the octanol phase to the concentration of the target compound dissolved in the aqueous phase in a two-phase system of octanol and water. Kow is measured at room temperature.
Kow = concentration of octanol phase/concentration of aqueous phase

In one embodiment, the solvent used in the present invention is classified as exemplified below.

**[Table 1]**

| Category | Compound | log Kow | Polarity |
|---|---|---|---|
| Protic | Propylene glycol | -1.4 | High polarity |
| | Methanol | -0.77 | |
| | Ethanol | -0.24 | |
| | Acetone | -0.24 | |
| Aprotic | Ethyl acetate | 0.73 | Medium polarity |
| | Diethyl ether | 0.89 | |
| | Benzyl alcohol | 1.1 | |
| | Cumyl alcohol* | 1.95 | |
| | Chloroform | 1.97 | |
| | Benzene | 2.13 | Nonpolarity |
| | Hexane | 3.9 | |

| | | | |
|---|---|---|---|
| Source: www.chem.ucla.edu/~bacher/General/30BL/tips/solvent.html *Source: MSDS | | | |

In this process, the discharged solid may be subjected to extraction with an aprotic solvent and water or an acid aqueous solution. This is because nicotine in the discharged solid can be transferred to the aqueous phase by water. Since nicotine in the discharged solid can be transferred, in the form of a salt, to the aqueous phase by acid, the water preferably contains an acid, that is, the water is preferably an acid aqueous solution. As the acid, an inorganic acid or an organic acid can be used, but sulfuric acid, citric acid, or oxalic acid is preferable from the viewpoint of stability of the nicotine salt and the like. The pH of the acid aqueous solution is preferably 4 or less, and more preferably 3 or less. When the pH exceeds 4, nicotine may not be sufficiently extracted. The lower limit of the pH is not limited, but is preferably 2 or more. The temperature at which this process is carried out is not limited, but is preferably 10 to 35°C, and more preferably 20 to 30°C. In this process, it is preferable that the discharged solid be subjected to solid-liquid extraction with an aprotic solvent, and then the organic phase obtained by the solid-liquid extraction be subjected to liquid-liquid extraction with water or an acid aqueous solution. After the solid-liquid extraction, an insoluble solid content may be removed by filtration or the like.

### (3) Process 3

In this process, the organic phase obtained in the process 2 is recovered. The organic phase is the organic phase obtained by the solid-liquid extraction or the organic phase obtained by the liquid-liquid extraction. The recovery method is not limited, and can be performed using, for example, a separatory funnel. If necessary, the aqueous phase may be washed with an aprotic solvent, and the washed solvent may be added to the organic phase. In this way, a solution of a tobacco extract containing CBT can be obtained. The aprotic solvent may be removed from the solution to prepare a concentrated solution, or the tobacco extract may be isolated. The method for removing the solvent is not limited, and for example, an evaporator can be used.

### (4) Purification process

The production method of the present invention may include a process of purifying the organic phase. Purification can be performed by subjecting the organic phase or a tobacco extract obtained by removing the solvent from the organic phase to normal phase chromatography using a mixed solvent of n-hexane and ethyl acetate as a mobile phase, and thereby obtaining a fraction of n-hexane and ethyl acetate (50 : 50) and a fraction of n-hexane and ethyl acetate (40 : 60) (process P1). Alternatively, purification can also be performed by adding methanol or ethanol to the organic phase or a tobacco extract obtained by removing the solvent from the organic phase to produce a precipitate, and obtaining a solution from which the precipitate has been removed (process P2). Further, the fractions obtained in the process P1, that is, a fraction of n-hexane and ethyl acetate (50 : 50) and a fraction of n-hexane and ethyl acetate (40 : 60) or the solution obtained in the process P2 may be subjected to reverse phase chromatography using water and methanol as a mobile phase (process P3). Through these processes, the content of CBT in the tobacco extract can be increased. In the present invention, CBT refers to α-CBT, β-CBT, or a combination thereof.

### 2. Tobacco extract

The tobacco extract obtained by the production method (hereinafter, also referred to as "tobacco extract of the present invention") contains CBT. Since the CBT imparts smoking flavor of tobacco, the tobacco extract of the present invention is useful as a tobacco flavoring agent. Further, the tobacco extract obtained through the purification process has a content of CBT of preferably 90% or more, more preferably 95% or more, and still more preferably 99% or more, and thus has high purity. The tobacco flavoring agent is a tobacco extract in one embodiment, and contains a tobacco extract and other components in another embodiment.

### 3. Tobacco material

A tobacco flavoring agent containing the tobacco extract of the present invention (hereinafter also referred to as "tobacco flavoring agent of the present invention") is useful as an additive to a tobacco material. Examples of the tobacco material include a tobacco sheet, a shredded tobacco, a cigarette paper, and a polysaccharide sheet. The tobacco material to which the tobacco flavoring agent of the present invention has been added is also referred to as "tobacco material of the present invention".

### (1) Tobacco sheet

The tobacco sheet is a sheet obtained by forming a composition containing aged tobacco leaves and the like into a sheet shape. The aged tobacco leaves used for the tobacco sheet are not particularly limited, and examples thereof include laminas and stems separated from tobacco leaves by stemming. The aged tobacco leaves refer to tobacco leaves that have undergone processing such as curing and long-term storage in a warehouse or the like. In the present invention, the "sheet" refers to a material having a pair of substantially parallel main surfaces and side surfaces. The tobacco sheet can be formed by a known method such as a papermaking method, a casting method, or a rolling method. Details of various tobacco sheets formed by such methods are disclosed in "Encyclopedia of Tobacco, Tobacco Academic Studies Center, March, 31, 2009". The embodiment in which the tobacco flavoring agent of the present invention is added to the tobacco sheet is not limited.

For example, the tobacco extract of the present invention is dissolved in a solvent to prepare a solution of the tobacco flavoring agent, and this may be sprayed onto or impregnated into a completed tobacco sheet. Alternatively, the tobacco flavoring agent of the present invention may be added to the tobacco sheet when the tobacco sheet is formed. For example, the papermaking method includes: separating aged tobacco leaves into a water extract and a residue through extraction of watersoluble components from the tobacco leaves; subjecting a mixture of the residue that has been defibrated and pulp to papermaking; and adding a concentrate of the water extract to a sheet obtained by papermaking. In this method, the tobacco flavoring agent of the present invention can be added to the water extract. The casting method includes: mixing water, pulp, a binder, a pulverized product of aged tobacco to prepare a mixture; and subjecting the mixture to casting. In this method, the tobacco flavoring agent of the present invention can be added to the mixture. The rolling method includes: mixing water, pulp, a binder, and a pulverized product of aged tobacco to prepare a mixture; supplying the mixture to a plurality of rolling rollers; and performing rolling. In this method, the tobacco flavoring agent of the present invention can be added to the mixture.

Further, as described in International Publication No. WO 2014/104078, a pulverized product of aged tobacco and a binder are mixed to prepare a mixture, the mixture is sandwiched between nonwoven fabrics, and the laminate is formed into a certain shape by heat welding, whereby a nonwoven fabric-shaped tobacco sheet can be obtained. In this method, the tobacco flavoring agent of the present invention can be added to the mixture.

The tobacco sheet may contain an aerosol-generating base material. The type of the aerosol-generating base material is not particularly limited, and various extracted substances from natural products or their constituent components can be selected depending on the application. Specific examples of the aerosol-generating base material may include polyhydric alcohols such as glycerin, propylene glycol, sorbitol, xylitol, and erythritol, triacetin, 1,3-butanediol, and mixtures thereof. The content of the aerosol-generating base material can be adjusted to various amounts depending on the form utilized in the tobacco product. For example, when the aerosol-generating base material is contained in the tobacco sheet, the content thereof is usually 5 wt% or more, preferably 10 wt% or more, and more preferably 15 wt% or more, and is usually 50 wt% or less, preferably 40 wt% or less, and more preferably 25 wt% or less, based on the total weight of the tobacco sheet, from the viewpoint of obtaining good flavor.

### (2) Shredded tobacco

Examples of the shredded tobacco include those obtained by shredding aged tobacco leaves into a predetermined size, those obtained by shredding the tobacco sheet into a predetermined size, and those obtained by mixing these. The size of the shredded tobacco is not limited, and examples thereof include those having a width of 0.5 to 2.0 mm and a length of 3 to 10 mm. The shredded tobacco having such a size is preferable in an embodiment in which an object to be filled described later is filled with the shredded tobacco. In addition, examples of the shredded tobacco include strand type shreds prepared by shredding processed tobacco leaves so as to have a width of 0.5 to 2.0 mm and a length longer than that of the shredded tobacco described above, preferably about the same length as that of the cigarette paper. The tobacco flavoring agent of the present invention may be added to a shredded tobacco, or may be added to a raw material before shredding.

The shredded tobacco may contain the aerosol-generating base material. When the aerosol-generating base material is contained in the shredded tobacco, the content thereof is usually 5 wt% or more, preferably 10 wt% or more, and more preferably 15 wt% or more, and is usually 50 wt% or less, preferably 40 wt% or less, and more preferably 25 wt% or less, based on the weight of the shredded tobacco, from the viewpoint of generating a sufficient amount of aerosol and obtaining good flavor.

### (3) Cigarette paper

A cigarette paper containing the tobacco flavoring agent can be prepared by, for example, spraying the tobacco flavoring agent of the present invention onto the cigarette paper, or impregnating the cigarette paper with the tobacco flavoring agent. Examples of the cigarette paper include paper containing pulp as a main component. Besides pulps made of wood pulp such as softwood pulp and hardwood pulp, the pulp may be a pulp obtained by mixing, with wood pulp, non-wood pulp generally used for a cigarette paper for tobacco articles, such as flax pulp, hemp pulp, sisal pulp, and esparto. These pulps may be used alone, or a plurality of types may be used in combination at any ratio. Further, the cigarette paper may be composed of one sheet, or may be composed of a plurality of sheets or more. The cigarette paper may be used in the form of a wrapped member in which a tobacco raw material such as shredded tobacco is wrapped with the cigarette paper. The cigarette paper can also be used as a material (for example, tipping paper) for wrapping the wrapped member together with other members such as a cooling member and a filter member. As the pulp, a chemical pulp obtained by a kraft cooking method, an acidic-neutral-alkali sulfite cooking method, a soda cooking method or the like, a ground pulp, a chemiground pulp, a thermomechanical pulp and the like can be used.

### (4) Polysaccharide sheet

The polysaccharide sheet is a sheet containing a polysaccharide as a main component, and the polysaccharide sheet can contain the tobacco flavoring agent of the present invention. The flavor inhalation article using the polysaccharide sheet containing the tobacco flavoring agent of the present invention can release sufficient flavor. Examples of the polysaccharide include carrageenan, agar, gellan gum, tamarind gum, psyllium seed gum, konjac glucomannan, carrageenan, locust bean gum, guar gum, agar, xanthan gum, gellan gum, tamarind gum, tara gum, konjac glucomannan, starch, cassia gum. and psyllium seed gum.

The polysaccharide sheet containing the tobacco flavoring agent of the present invention can be used for a combustion type tobacco flavor inhalation article and a non-combustion type tobacco flavor inhalation article. In the former embodiment, for example, the polysaccharide sheet as disclosed in Japanese Patent No. 5481574 can be used. In this embodiment, the content of the tobacco flavoring agent of the present invention can be preferably 10 wt% or more, more preferably 18 wt% or more, still more preferably 60 wt% or more, and particularly preferably 70 wt% or more, based on the weight of the sheet. The polysaccharide sheet can be prepared by a method in which a polysaccharide and water are mixed and heated to prepare an aqueous solution of a polysaccharide, a flavor and an emulsifier are added to the aqueous solution, and the mixture is kneaded and emulsified. As the emulsifier, a known emulsifier can be used.

In the latter embodiment, the polysaccharide sheet as described in PCT/JP 2019/20136 can be used. In this embodiment, agar is particularly preferably used as the polysaccharide. The content of the agar is preferably 10 to 50 wt%, more preferably 15 to 45 wt% based on the weight of the sheet. In addition, the content of the tobacco flavoring agent of the present invention in the polysaccharide sheet can be 35 to 80 wt% based on the weight of the sheet.

In this embodiment, it is preferable to use a saccharide compound selected from the group consisting of sugars and sugar alcohols. Examples of the "sugar" include glucose, sucrose, fructose, xylose, galactose, mannose, maltose, trehalose, lactose, and raffinose. Examples of the "sugar alcohol" include sorbitol which is an alcohol obtained by reducing the carbonyl group of a sugar to a hydroxyl group. The content of the compound is preferably 10 wt% or more, more preferably 10 to 500 wt%, still more preferably 10 to 300 wt%, and still more preferably 10 to 200 wt%, based on the weight of the agar. In this embodiment, an emulsifier is preferably used. As the emulsifier, a known emulsifier can be used, and the content thereof is preferably 0.5 to 10 wt% and more preferably 1.0 to 8.0 wt% based on the weight of the agar.

The polysaccharide sheet in this embodiment can be produced by kneading raw materials containing agar, a saccharide compound, a flavor, and an emulsifier in water to prepare a raw material slurry, extending the raw material slurry on a base material, and drying the slurry.

### 4. Tobacco flavor inhalation article

In the present invention, the "flavor inhalation article" refers to an article for a user to inhale flavor. Among flavor inhalation articles, those having tobacco or a component derived from the tobacco are referred to as "tobacco flavor inhalation article". The tobacco flavor inhalation article is roughly classified into "combustion type tobacco flavor inhalation articles" (also simply referred to as "smoking article") that generate flavor by combustion, and "non-combustion type tobacco flavor inhalation articles" that generate flavor without combustion. Further, the non-combustion type tobacco flavor inhalation article is roughly classified into "non-combustion heating type tobacco flavor inhalation articles" that generate flavor by heating and "non-combustion non-heating type tobacco flavor inhalation articles" that generate flavor without heating. The tobacco flavoring agent of the present invention is suitable for the non-combustion heating type tobacco flavor inhalation article or the non-combustion non-heating type tobacco flavor inhalation article. In addition, a combination of a device (a heating apparatus, an atomizing apparatus, or the like) for generating aerosol and the non-combustion heating type tobacco flavor inhalation article is also particularly referred to as a non-combustion heating type tobacco flavor inhalation system.

### (1) Non-combustion heating type tobacco flavor inhalation article

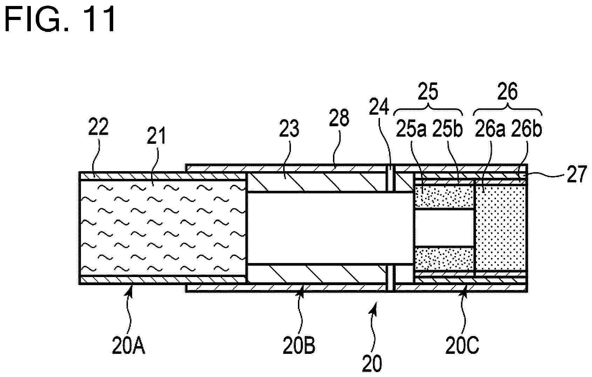

FIG. 11 illustrates one embodiment of a non-combustion heating type tobacco flavor inhalation article. As illustrated in the drawing, a non-combustion heating type tobacco flavor inhalation article 20 includes a tobacco rod part 20A, a cylindrical cooling part 20B having perforations on the periphery thereof, and a filter part 20C. The non-combustion heating type tobacco flavor inhalation article 20 may have other members. The axial length of the non-combustion heating type tobacco flavor inhalation article 20 is not limited, but is preferably 40 to 90 mm, more preferably 50 to 75 mm, and still more preferably 50 to 60 mm or less. In addition, the circumferential length of the non-combustion heating type tobacco flavor inhalation article 20 is preferably 16 to 25 mm, more preferably 20 to 24 mm, and still more preferably 21 to 23 mm. For example, an embodiment can be exemplified in which the length of the tobacco rod part 20A is 20 mm, the length of the cooling part 20B is 20 mm, and the length of the filter part 20C is 7 mm. The length of each member can be appropriately changed according to production suitability, required quality, and the like. FIG. 11 illustrates an embodiment in which a first segment 25 is disposed, but only a second segment 26 may be disposed downstream of the cooling part 20B without disposing the first segment 25.

### 1) Tobacco rod part 20A

In the tobacco rod part 20A, as a tobacco filler 21, a shredded tobacco or a tobacco sheet containing the tobacco flavoring agent of the present invention can be used. A method for filling the tobacco filler 21 into a cigarette paper 22 is not particularly limited, but for example, the tobacco filler 21 may be wrapped with the cigarette paper 22, or the tobacco filler 21 may be filled into the cylindrical cigarette paper 22. When the shape of the tobacco has a longitudinal direction like a rectangular shape, the tobacco filer may be filled so that each longitudinal direction is an unspecified direction in the cigarette paper 22, or may be filled so as to be aligned in the axial direction of the tobacco rod part 20A or aligned in a direction orthogonal thereto. As the cigarette paper 22, a cigarette paper containing the tobacco flavoring agent of the present invention described above can also be used. When the tobacco rod part 20A is heated, the tobacco component, the aerosol-generating base material and water contained in the tobacco filler 21 are vaporized and provided for inhalation.

### 2) Cooling part 20B

The cooling part 20B is preferably formed of a cylindrical member. The cylindrical member may be, for example, a paper tube 23 obtained by processing a cardboard into a cylindrical shape. The cooling part 20B may also be formed by a sheet of thin material that is wrinkled, then creased, gathered, or folded to form a channel. As such a material, for example, a sheet material selected from the group consisting of polyethylene, polypropylene, polyvinyl chloride, polyethylene terephthalate, polylactic acid, cellulose acetate, and aluminum foil can be used. The total surface area of the cooling part 20B is appropriately prepared in consideration of cooling efficiency, and can be set to, for example, 300 to 1,000 mm²/mm. The cooling part 20B is preferably provided with perforations 24. Due to the presence of the perforations 24, outside air is introduced into the cooling part 20B at the time of inhalation. As a result, the aerosol vaporization component generated by heating the tobacco rod part 21A comes into contact with the outside air, and the temperature thereof is lowered, so that the aerosol is liquefied and formed. The diameter (length across the perforation) of the perforation 24 is not particularly limited, but may be, for example, 0.5 to 1.5 mm. The number of perforations 24 is not particularly limited, and may be one or two or more. For example, a plurality of perforations 24 may be provided on the periphery of the cooling part 20B.

The cooling part 20B can have a rod shape having an axial length of, for example, 7 to 28 mm The axial length of the cooling part 20B can be set to, for example, 18 mm. The cooling part 20B has a substantially circular axial cross-section, and can have a diameter of 5 to 10 mm. The diameter of the cooling part can be, for example, about 7 mm.

### 3) Filter part 20C

The configuration of the filter part 20C is not particularly limited, but may be composed of one or a plurality of filling layers. The outside of the filling layer may be wrapped with one or more sheets of cigarette paper. The air-flow resistance of the filter part 20C can be appropriately changed depending on the amount, material, and the like of the filler filled in the filter part 20C. For example, when the filler is cellulose acetate fibers, the air-flow resistance can be increased by increasing the amount of cellulose acetate fibers filled in the filter part 20C. When the filler is cellulose acetate fibers, the filling density of the cellulose acetate fibers can be 0.13 to 0.18 g/cm³. The air-flow resistance is a value measured by an air-flow resistance measuring device (trade name: SODIMAX, manufactured by SODIM).

The circumferential length of the filter part 20C is not particularly limited, but is preferably 16 to 25 mm, more preferably 20 to 24 mm, and still more preferably 21 to 23 mm. The axial length of the filter part 20C (horizontal direction in FIG. 11) can be selected to be 4 to 10 mm so that the air-flow resistance thereof is 15 to 60 mmH₂O/seg. The axial length of the filter part 20C is preferably 5 to 9 mm, and more preferably 6 to 8 mm. The shape of the cross section of the filter part 20C is not particularly limited, and can be, for example, a circle, an ellipse, a polygon, or the like. Further, a breakable capsule containing a flavor, flavor beads, and a flavor may be directly added to the filter part 20C.

The filter part 20C may include a center hole part as the first segment 25. The center hole part is composed of a first filling layer 25a having one or a plurality of hollow portions, and an inner plug wrapper (inner cigarette paper) 25b covering the filling layer. The center hole part has a function of increasing the strength of a mouthpiece part. The center hole part may not have the inner plug wrapper 25b, and the shape of the center hole part may be maintained by thermal molding. The filter part 20C may include the second segment 26. The second segment 26 is composed of a second filling layer 26a and an inner plug wrapper (inner cigarette paper) 26b covering the filling layer. The second filling layer 26b can be, for example, a rod having an inner diameter ϕ of 5.0 to ϕ 1.0 mm made by filling the cellulose acetate fibers densely and then curing, wherein a plasticizer containing triacetin of 6 to 20 wt% (based on the weight of cellulose acetate) has been added to the cellulose acetate fibers. Since the second filling layer has a high filling density of fibers, air and aerosol flow only through the hollow portion at the time of inhalation, and hardly flow in the second filling layer. Since the second filling layer inside the center hole segment is a fiber filling layer, the touch feeling from the outside at the time of use hardly causes discomfort to the user.

The first filling layer 25b and the second filling layer 26b are connected by an outer plug wrapper (outer cigarette paper) 27. The outer plug wrapper 27 can be, for example, cylindrical paper. The tobacco rod part 20A, the cooling part 20B, and the first filling layer 25a and second filling layer 26b which have been connected are connected by a mouthpiece lining paper 28. The connection of these members can be made by, for example, applying a glue such as a vinyl acetate-based glue to the inner surface of the mouthpiece lining paper 28, and wrapping the mouthpiece lining paper 28 around the three members. These members may be connected by a plurality of times with a plurality of sheets of lining papers.

### 4) Non-combustion heating type tobacco flavor inhalation system

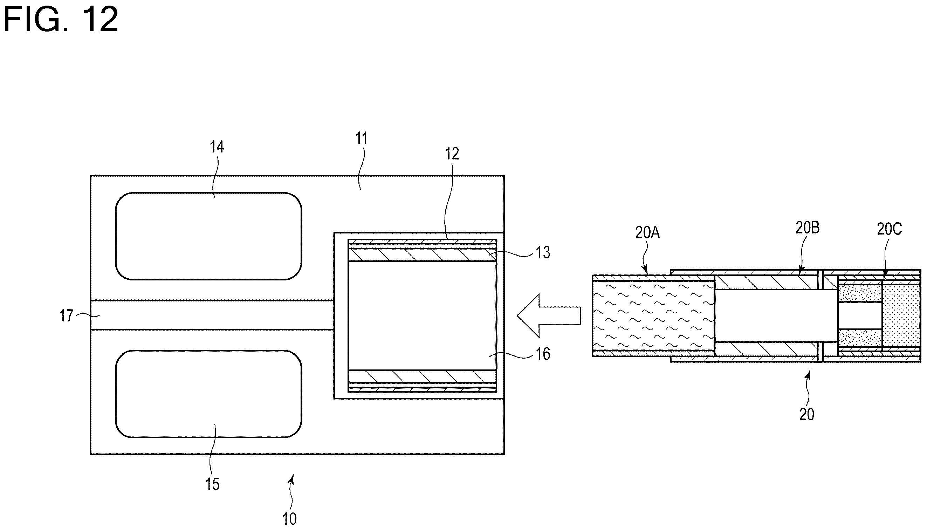

A combination of a non-combustion heating type tobacco flavor inhalation article and a heating device for generating aerosol is particularly also referred to as a non-combustion heating type tobacco flavor inhalation system. An example of the system is illustrated in FIG. 12. In the drawing, the non-combustion heating type tobacco flavor inhalation system includes the non-combustion heating type tobacco flavor inhalation article 20 and a heating device 10 that heats the tobacco rod part 20A from the outside.

The heating device 10 includes a body 11, a heater 12, a metal tube 13, a battery unit 14, and a control unit 15. The body 11 has a cylindrical recess 16, and the heater 12 and the metal tube 13 are disposed at positions corresponding to the tobacco rod part 20A to be inserted into the recess. The heater 13 can be a heater by electric resistance, and power is supplied from the battery unit 14 according to an instruction from the control unit 15 that performs temperature control, and the heater 12 is heated. The heat generated from the heater 12 is transferred to the tobacco rod part 20A through the metal tube 13 having high thermal conductivity. In the drawing, an embodiment is illustrated in which the heating device 10 heats the tobacco rod part 20A from the outside, but the heating device may heat the tobacco rod part 20A from the inside. The heating temperature of the heating device 10 is not particularly limited, but is preferably 400°C or lower, more preferably 150 to 400°C, and still more preferably 200 to 350°C. The heating temperature indicates the temperature of the heater of the heating device 10.

### (3) Non-combustion non-heating type tobacco flavor inhalation article

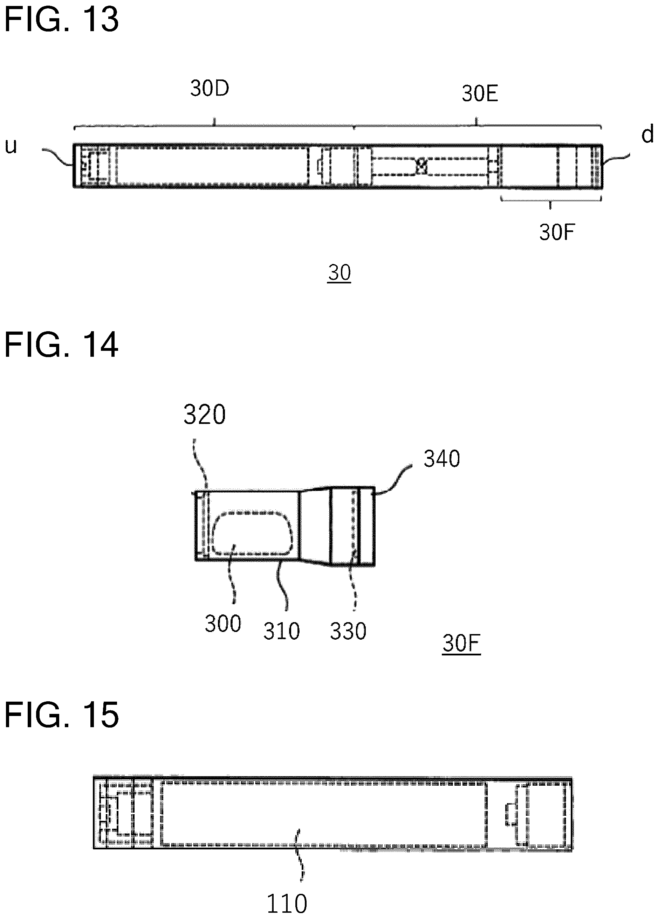

FIG. 13 illustrates one embodiment of a non-combustion non-heating type tobacco flavor inhalation article. A non-combustion non-heating type tobacco flavor inhalation article 30 includes a power supply unit 30D, a cartridge 30E, and a tobacco capsule 30F. The non-combustion non-heating type tobacco flavor inhalation article 30 has a shape extending from a non-inhalation end u (upstream) toward an inhalation end d (downstream). The cartridge 30E is detachable from the power supply unit 30D. The tobacco capsule 30F is detachable from the cartridge 30E.

### 1) Tobacco capsule

FIG. 14 illustrates an example of the tobacco capsule 30F. As shown in the drawing, the tobacco capsule 30F is a tobacco rod part, and has a flavor source 300 therein. The flavor source 300 contains a tobacco material (tobacco material of the present invention) containing the tobacco flavoring agent of the present invention. The tobacco capsule 30F is connected to the cartridge 30E. Specifically, a part of the tobacco capsule 30F is accommodated in the cartridge 30E.

The tobacco capsule 30F includes a container 310 that accommodates the flavor source 300, a mesh body 320, a nonwoven fabric 330, and a cap 340. The aerosol atomized by an atomizer 220 to be described later is introduced into the container 310 through the mesh body 320 and comes into contact with the flavor source 300, whereby flavor is imparted to the aerosol. Thereafter, the aerosol is inhaled into the user through the nonwoven fabric 330. As described above, in the non-combustion non-heating type tobacco flavor inhalation article 30, flavor can be imparted to aerosol without heating the flavor source 300. In addition, substantially no aerosol is generated from the flavor source 300.

In the flow direction of aerosol, the length of the tobacco capsule 30F (container 310) is preferably 40 mm or less, and more preferably 25 mm or less. In the flow direction of aerosol, the length is preferably 1 mm or more, and more preferably 5 mm or more. In the direction orthogonal to the flow direction of aerosol, the maximum length of the container 310 of the tobacco capsule 30F (container 310) is preferably 20 mm or less, and more preferably 10 mm or less. In addition, in the direction orthogonal to the flow direction of aerosol, the maximum length of the tobacco capsule 30F (container 310) is preferably 1 mm or more, and more preferably 3 mm or more.

The flavor source 300 containing tobacco is composed of raw material pieces that impart flavor to aerosol. The lower limit of the size of the raw material piece is preferably 0.2 to 1.2 mm, and more preferably 0.2 to 0.7 mm. As the size of the raw material piece constituting the flavor source 300 decreases, the specific surface area increases, so that the smoking flavor components are easily released. As the raw material piece constituting the flavor source 300, a shredded tobacco which is the tobacco material of the present invention, a formed body obtained by forming the tobacco raw material of the present invention into particles, or the like can be used. The flavor source 300 may contain a flavor of plants (for example, mint, herb, and the like) other than tobacco, such as menthol. Further, the flavor source 300 containing tobacco may contain a taste material. Examples of the taste material include materials exhibiting sweetness, sourness, saltiness, umami, bitterness, astringency taste, body taste, pungency, harsh taste, astringency, and the like. Examples of the material exhibiting sweetness include saccharides, sugar alcohols, and sweeteners. Examples of the saccharide include monosaccharides, disaccharides, oligosaccharides, and polysaccharides. Examples of the sweetener include natural sweeteners and synthetic sweeteners.

The raw material pieces are obtained, for example, by sieving in accordance with JIS Z 8801 using a stainless steel sieve in accordance with JIS Z 8815. For example, the raw material pieces are sieved over 20 minutes using a stainless steel sieve having a mesh opening of 0.71 mm by a dry type mechanical shaking method, to obtain raw material pieces which have passed through the stainless steel sieve having a mesh opening of 0.71 mm. Subsequently, the raw material pieces are sieved over 20 minutes using a stainless steel sieve having a mesh opening of 0.212 mm by a dry type mechanical shaking method, to remove raw material pieces which have passed through the stainless steel sieve having a mesh opening of 0.212 mm. That is, the raw material pieces constituting the flavor source 300 are raw material pieces that have passed through a stainless steel sieve (mesh opening = 0.71 mm) defining the upper limit and have not passed through a stainless steel sieve (mesh opening = 0.212 mm) defining the lower limit. Therefore, the lower limit of the size of the raw material piece constituting the flavor source 300 is defined by the mesh opening of the stainless steel sieve that defines the lower limit. The upper limit of the size of the raw material piece constituting the flavor source 300 is defined by the mesh opening of the stainless steel sieve that defines the upper limit.

The filling amount of the flavor source 300 to be accommodated in the container 310 is preferably 300 mg or more, and more preferably 350 mg or more, from the viewpoint of increasing the volatilization amount of nicotine during smoking.

### 2) Power supply unit

An example of the power supply unit 30D is illustrated in FIG. 15. The power supply unit 30D includes a battery 110. The battery 110 may be a disposable battery or a rechargeable battery. The initial value of the output voltage of the battery 110 is preferably in a range of 1.2 V or more and 4.2 V or less. The battery capacity of the battery 110 is preferably in a range of 100 mAh or more and 1,000 mAh or less.

### 3) Cartridge

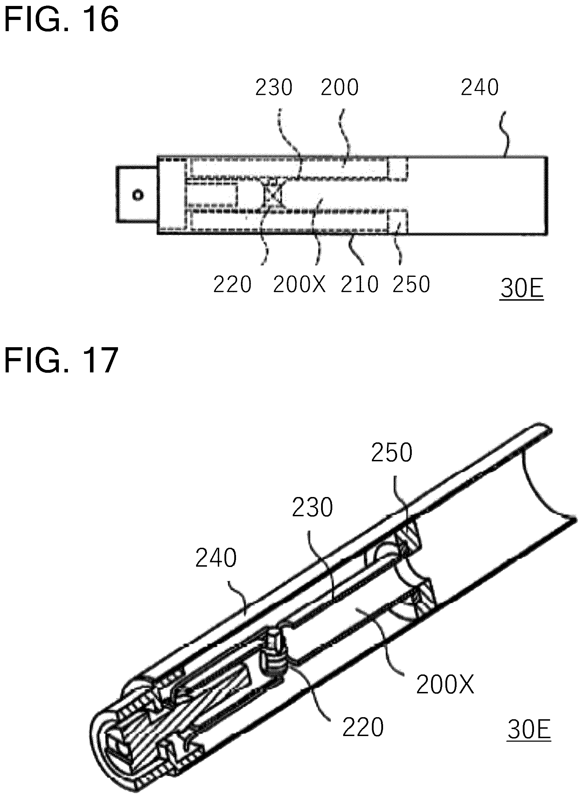

An example of the cartridge 30E is illustrated in FIGs. 16 and 17. FIG. 16 is a cross-sectional view of an example of the cartridge 30E, and FIG. 17 is a view illustrating an internal structure of the cartridge 30E. The cartridge 30E has a reservoir 210, an atomizer 220, a flow path forming body 230, an outer frame 240, and an end cap 250. The cartridge 30D has a first flow path 200X disposed downstream of the atomizer 220 as an aerosol flow path.

The reservoir 210 stores an aerosol source 200. The reservoir 210 is located around the flow path forming body 230 in a cross section orthogonal to the flow direction of aerosol (direction from the non-inhalation end to the inhalation end (upstream to downstream)). The reservoir 210 is located in a gap between the flow path forming body 230 and the outer frame 240. The reservoir 210 is made of, for example, a porous body such as a resin web or cotton. Further, the reservoir 210 may be composed of a tank that accommodates the liquid aerosol source 200. Examples of the aerosol source 200 include glycerin and propylene glycol.

The atomizer 220 atomizes the aerosol source 200 without combustion by the electric power supplied from the battery 110. The atomizer 220 is formed of heating wires (coils) wound at a predetermined pitch. The atomizer 220 is preferably formed of heating wires having a resistance value in a range of 1.0 to 3.0 Ω. The predetermined pitch is equal to or larger than a value at which the heating wires do not contact each other, and is preferably a smaller value. The predetermined pitch is preferably, for example, 0.40 mm or less. The predetermined pitch is preferably constant to stabilize the atomization of the aerosol source 200. The predetermined pitch is an interval between the centers of heating wires adjacent to each other.

The flow path forming body 230 has a cylindrical shape and forms the first flow path 200X extending along the flow direction of aerosol. The outer frame 240 has a cylindrical shape and accommodates the flow path forming body 230. The outer frame 240 extends downstream of the end cap 250 and accommodates a part of the tobacco capsule 30F. The end cap 250 is a cap that closes the gap between the flow path forming body 230 and the outer frame 240 from the downstream side. The end cap 250 suppresses a situation in which the aerosol source 200 stored in the reservoir 210 leaks to the tobacco capsule 30E side.

### 5. Smokeless tobacco

The smokeless tobacco is a product that contains a flavor source and allows the user to taste a flavor derived from the flavor source by directly taking the product in the nasal cavity or oral cavity. As the flavor source contained in the smokeless tobacco, the tobacco material of the present invention can be used. As the smokeless tobacco, snuff tobacco and chewing tobacco are known.

### EXAMPLES

### [Example 1]

A discharged solid obtained from the expansion treatment process of a tobacco raw material using supercritical carbon dioxide was prepared. Specifically, the treatment as shown in FIG. 18 was performed to impregnate a tobacco raw material with carbon dioxide in a supercritical state, the tobacco raw material in a dry ice state was taken out, and then the tobacco raw material was air-dried at once to remove carbon dioxide. A discharged solid (Dust) resulting from solidification of gum components and shredded fine powder separated from high-temperature carbon dioxide gas (Tail gas) and carbon dioxide discharged at that time was obtained as the discharged solid. First, 150 g of the discharged solid was weighed, and put into a 2,500 ml sealed stainless steel container. Next, 1,500 ml of ethyl acetate (for high-performance liquid chromatography, FUJIFILM Wako Pure Chemical Corporation) was added to the discharged solid, and extraction was then performed for 3 hours in a warm bath at 40°C under hermetically stirring. After the extraction, the ethyl acetate solution and the extraction residue were separated with a stainless steel mesh having a mesh opening of 250 µm to obtain about 1,300 ml of an ethyl acetate solution. A 0.1% sulfuric acid aqueous solution (pH = 3) was prepared in advance, and the 0.1% sulfuric acid aqueous solution and the previously obtained ethyl acetate solution were mixed at a solution ratio of 5 : 3 to obtain a 800 ml of a mixed solution. Further, 50 g of salt was added to the mixed solution, and the mixture was sufficiently shaken in a separatory funnel to perform a liquid-liquid extraction operation. At this time, tobacco alkaloid components mainly composed of nicotine migrated to the sulfuric acid aqueous solution in the lower layer, and hydrophobic active ingredients of the tobacco leaves migrated to the organic phase in the upper layer. After sufficient leaving, the organic phase was taken out, and about 50 g of anhydrous sodium sulfate was added and stirred to perform a dehydration operation in the organic phase. Insoluble matters were removed by filtration with filter paper. Further, ethyl acetate was removed under reduced pressure with a rotary evaporator (manufactured by Nihon Buchi) to obtain 6.8 g (yield: 4.5%) of a dried solid. The dried solid was dissolved in the same solvent as the solvent used in the extraction to prepare a solution having a concentration of the dried solid of 4 wt%. This solution was subjected to GC/MS analysis under the following conditions.
Gas chromatography with mass spectrometer (GC/MS)
Apparatus: 7890A/5975C GC/MSD manufactured by Agilent Technologies, Inc.
GC conditions
Column: HP-5MS (manufactured by Agilent Technologies, Inc.)
Inner diameter 0.25 mm × length 30 m, film thickness: 0.25 µm
Injection volume: 1 µl
Injection mode: split (10 : 1)
Inlet temperature: 270°C
Septum purge flow rate: 5 ml/min
Carrier gas: helium (He)
Column flow rate: 1 ml/min (constant flow mode)
Oven temperature: 40°C (3 min) -4°C/min -280°C (20 min)
Transfer line temperature: 280°C
MS conditions
Solvent waiting time: 4 min
Ionization method: electron impact ionization (EI method), 70 eV
Ion source temperature: 230°C
Quadrupole temperature: 150°C
Measurement mode: scan
MS scan range: m/z 26 to 450
Threshold: 50
Sampling rate: 2

### [Comparative Example 1]

An amount of 5.4 g of a dried solid (yield: 3.6%) was obtained in the same manner as in Example 1 except that shreds before expansion were used in place of the discharged solid, and was analyzed under the same conditions as in Example 1.

Single ions (m/z 83) contained in the MS pattern of CBT were extracted from the total ion chromatogram obtained by the GC/MS analysis of the solid and the shreds before expansion, and the peak area values thereof were compared. The results show that the CBT peak area value of the solid was about 8 times larger than that of the shreds before expansion. Further, it can be said that the solid (unnecessary substance discharged from the expansion process) is suitable as a sample for extracting the CBT for effective use.

### [Example 2 and Comparative Example 2] study on organic solvent

The following were prepared as organic solvents used in the extraction process.
Example 2: n-hexane, chloroform, ethyl acetate
Comparative Example 2: acetone, methanol

Five 100 ml screw tubes were prepared, and about 5 g of the same discharged solid as used in Example 1 was weighed out into each screw tube, and 50 ml of each organic solvent was further added and mixed well, and then the screw tube was allowed to stand at normal temperature for a whole day and night. The mixed solution was filtered with filter paper, and anhydrous sodium sulfate was added to the filtrate in a small amount to perform dehydration. Then, the resultant was filtered again with filter paper to remove the organic solvent under reduced pressure. The obtained dried solid was dissolved in the same solvent as the solvent used in the extraction, to obtain a solution having a concentration of the dried solid of 4 wt%. Each solution was subjected to GC/MS analysis under the conditions shown in Example 1.

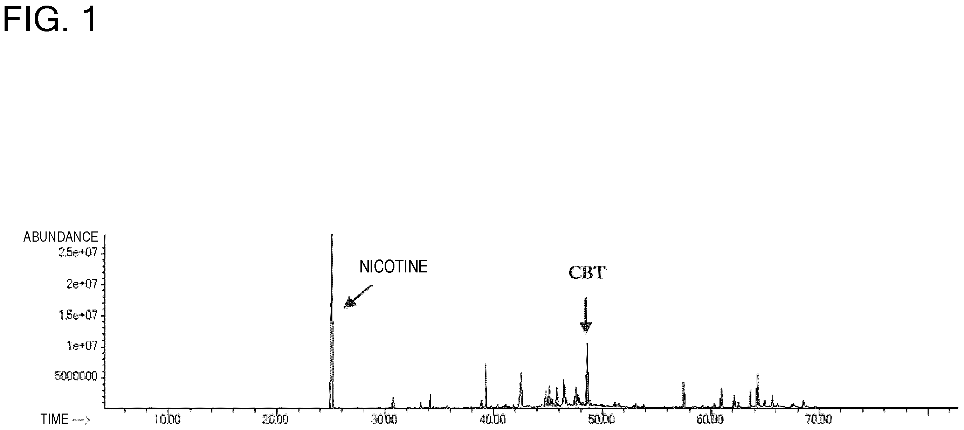

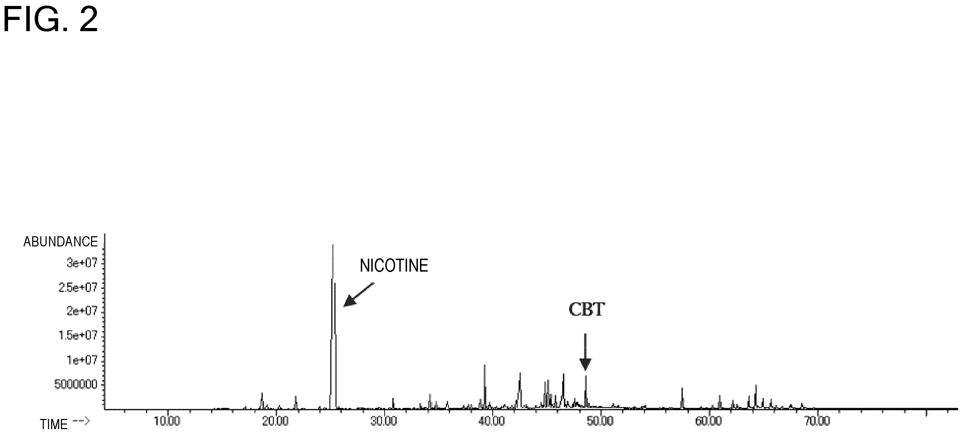

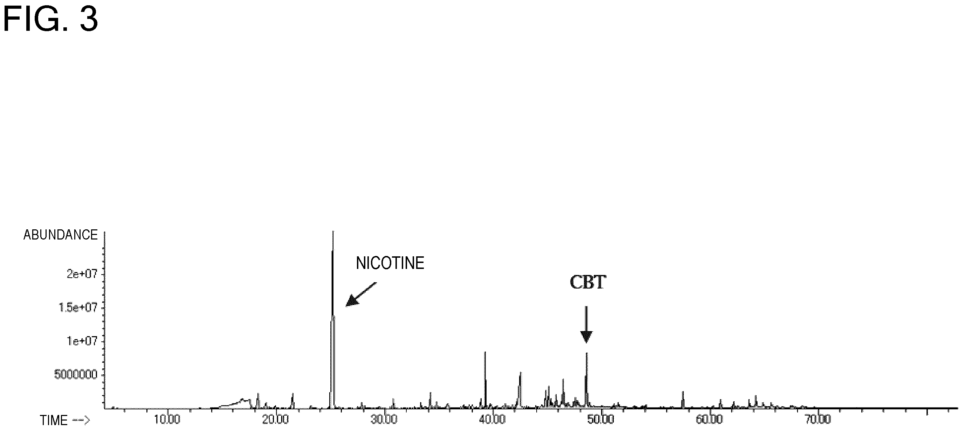

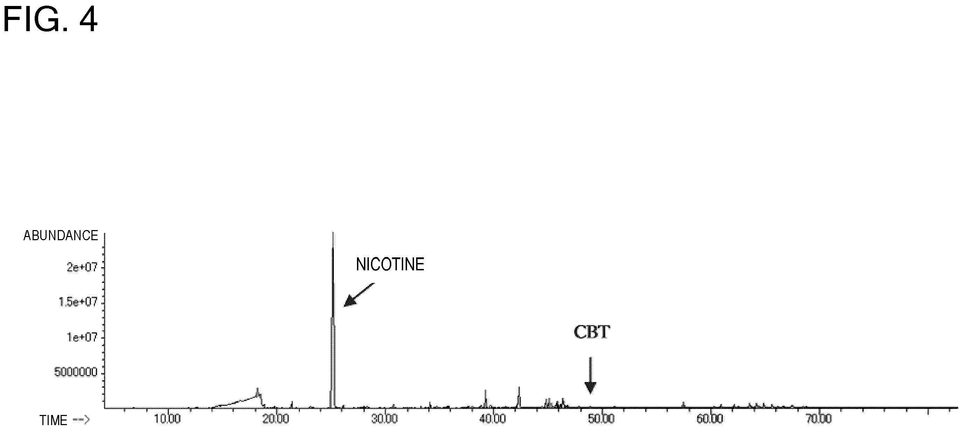

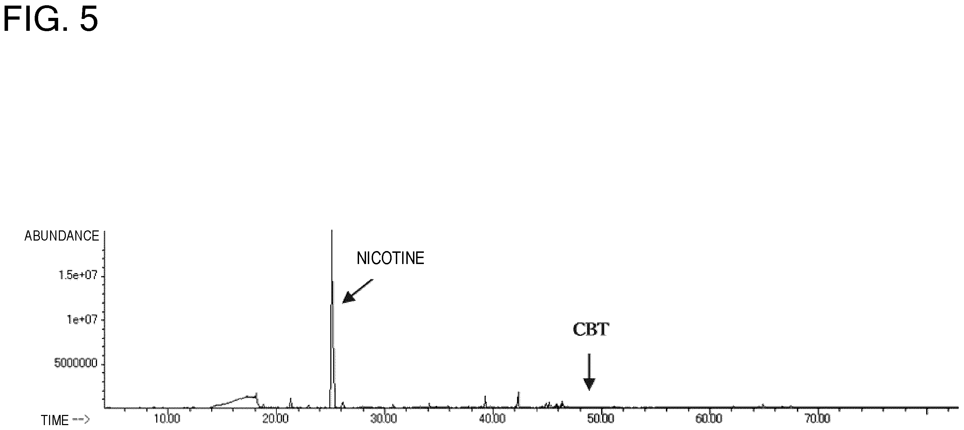

FIGs. 1 to 5 show total ion chromatograms obtained by GC/MS analysis of the n-hexane solution, the chloroform solution, the ethyl acetate solution, the acetone solution, and the methanol solution. The chromatograms in FIGs. 1 to 5 show that when solvent extraction was performed with n-hexane, chloroform, or ethyl acetate, CBT (retention time: 48.6 min) was extracted from the solid. In the extraction with chloroform or ethyl acetate, the presence of peaks of other components (24 minutes before the retention time) was confirmed. Among three types of solvents with which desired CBT can be extracted, n-hexane including a few peaks of unnecessary components as much as possible was found to be preferable as an extraction solvent. When the extraction process was performed with a polar solvent such as acetone or methanol, CBT was hardly extracted from the solid.

### [Example 3]

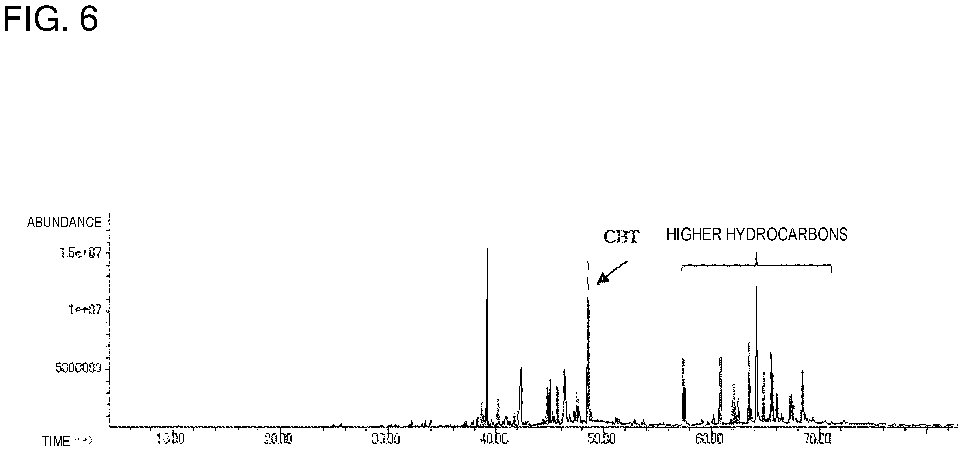

First, 300 g of the same discharged solid as used in Example 1 was weighed and put into a 2,500 ml sealed stainless steel container. Next, 1,500 ml of n-hexane (for high-performance liquid chromatography, FUJIFILM Wako Pure Chemical Corporation) was added to the discharged solid, and extraction was then performed for 3 hours in a warm bath at 40°C under hermetically stirring. After the extraction, the n-hexane solution and the extraction residue were separated with a stainless steel mesh having a mesh opening of 250 µm to obtain about 1,200 ml of an n-hexane solution. A 0.1% sulfuric acid aqueous solution was prepared in advance, and the 0.1% sulfuric acid aqueous solution and the n-hexane solution previously obtained were mixed at a solution ratio of 5 : 3 to obtain 800 ml of a mixed solution. Further, 50 g of salt was added to the mixed solution, and the mixture was sufficiently shaken in a separatory funnel to perform a liquid-liquid extraction operation. At this time, alkaloid components represented by nicotine migrated to the sulfuric acid aqueous solution in the lower layer, and hydrophobic active ingredients of the tobacco leaves migrated to the organic phase in the upper layer. After sufficient leaving, the organic phase was taken out, and about 50 g of anhydrous sodium sulfate was added and stirred to perform a dehydration operation in the organic phase. Insoluble matters were removed by filtration with filter paper. Further, n-hexane was removed under reduced pressure with a rotary evaporator (manufactured by Nihon Buchi) to obtain 6.9 g of a dried solid (yield: 2.3%). The dried solid was dissolved in the same solvent as the solvent used in the extraction to prepare a solution having a concentration of the dried solid of 4 wt%. This was subjected to GC/MS analysis under the same conditions as in Example 1 (FIG. 6). The chromatogram in FIG. 6 shows the presence of CBT (retention time: 48.6 min) and removal of alkaloids represented by nicotine (retention time: 24.6 min) by the liquid-liquid extraction operation.

### [Example 4]

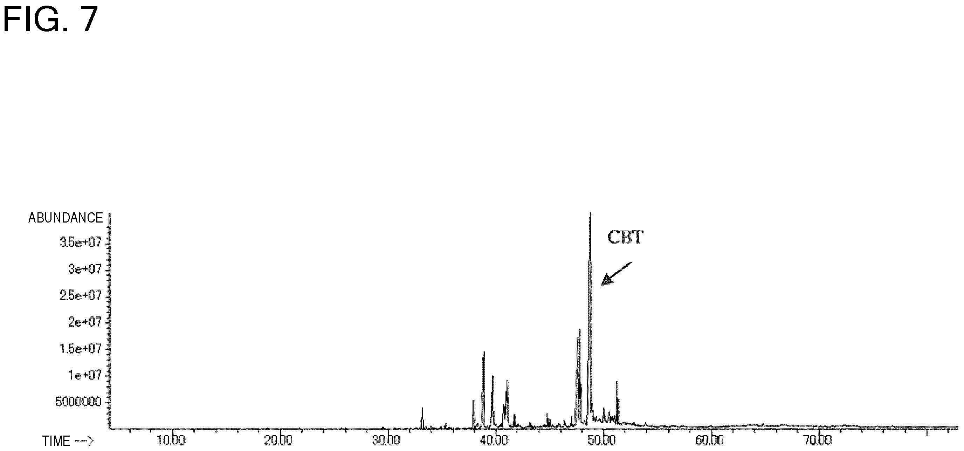

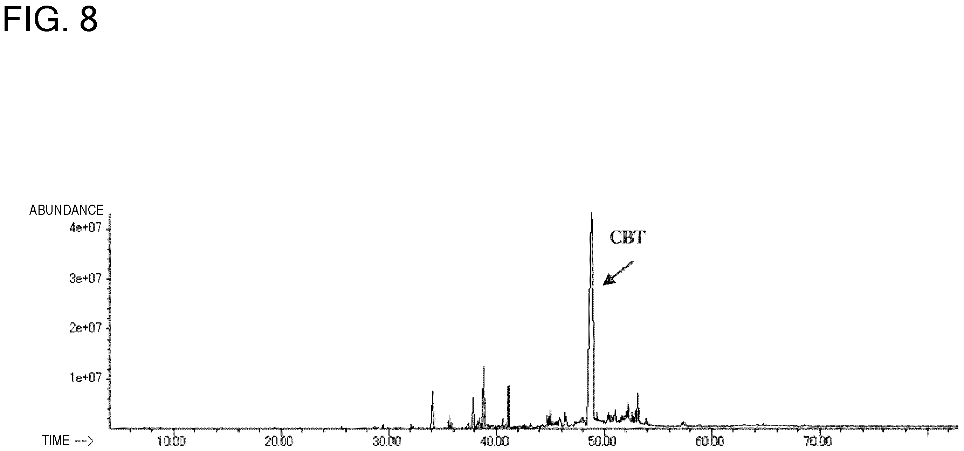

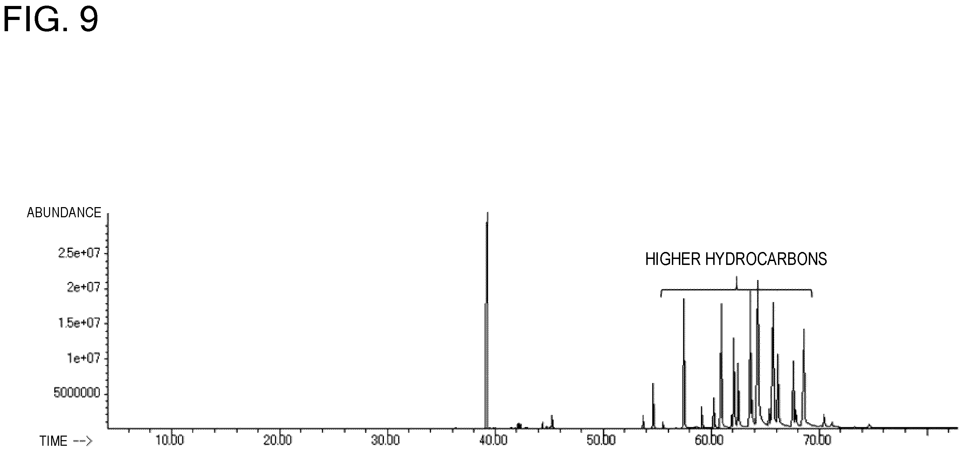

Component fractionation using normal phase column chromatography was performed on the dried solid containing CBT obtained in Example 3, from which alkaloids had been removed. To a glass chromatography column packed with 60 g of Wakosil C-300 (manufactured by FUJIFILM Wako Pure Chemical Corporation) mixed with n-hexane, 3.3 g of a solid dissolved in n-hexane was added. The mixture was sequentially developed with a mixed solvent of n-hexane and ethyl acetate (including single n-hexane and ethyl acetate). The resulting eluate was subjected to GC/MS analysis under the conditions shown in Example 1, and as a result, fractions of n-hexane and ethyl acetate 50 : 50 (FIG. 7) and 40 : 60 (FIG. 8) were confirmed as fractions containing CBT. Higher hydrocarbons (retention time: 57.4 to 68.4 min) other than CBT were found to elute with the first n-hexane single solvent (FIG. 9).

### [Example 5]

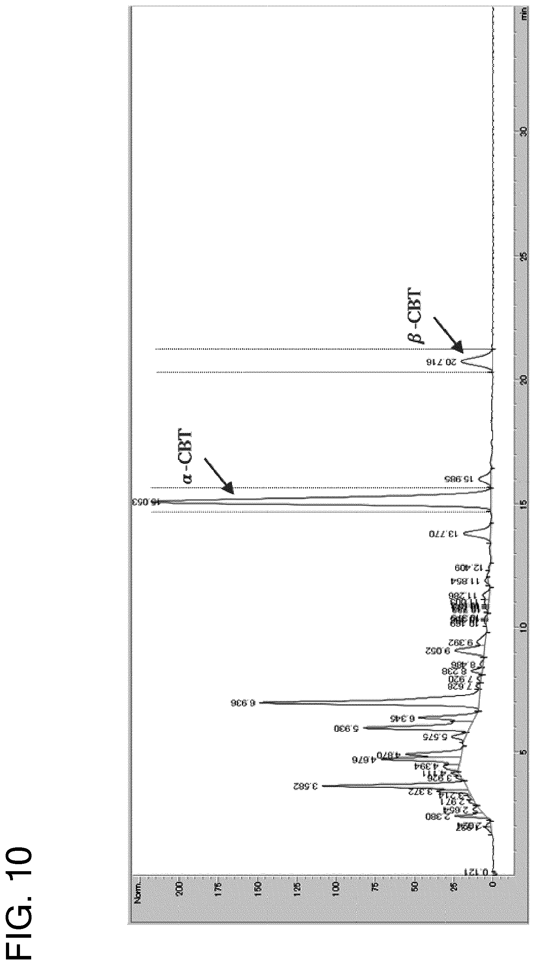

In order to isolate and purify CBT from the fractions containing CBT (a mixture of eluates of n-hexane and ethyl acetate (50 : 50) and n-hexane and ethyl acetate (40 : 60)) obtained in Example 4, reverse phase HPLC was performed as follows. A sample to be subjected to HPLC (1260 Infinity, manufactured by Agilent Technologies, Inc.) was treated with Sep-Pak Vac C18 (manufactured by Waters Corporation), and a sample from which coexisting low-polarity components such as a dye had been removed was used. The treated sample (500 µL) was injected. In the HPLC, isocratic elution was performed at a flow rate of 5 mL/min using water and MeOH (20/80) as a mobile phase, and YMC-Pack ODS-A/S -5 µm/12 nm, manufactured by YMC Co., Ltd. as a column. A diode array detector was used as the detector, and the detection wavelength was 214 nm. Then, the signal detected at 214 nm was fractionated into a fraction collector. When the detection time was confirmed using standard samples of α-CBT and β-CBT, α-CBT was detected at 15.0 min, and β-CBT was detected at 20.7 min. Therefore, each fraction corresponding to the above detection time (in the broken lines in FIG. 10) was recovered, and each sample was concentrated and dried to obtain α-CBT and β-CBT as white powders. In addition, from the total ion chromatograms obtained by GC/MS analysis, the peak area values of the dried solid obtained in Example 3 and the fractions containing CBT obtained above were compared, and it was confirmed that the yield of CBT was 71.5% (α-CBT: 57.6%, β-CBT: 13.9%), and CBT was obtained with high efficiency from the waste in the expansion process of tobacco.

According to the present invention, it is apparent that an extract containing CBT can be efficiently produced from the discharged solid obtained by the expansion treatment of a tobacco raw material by a simple method.

### [Example 6] Confirmation of effect with non-combustion type tobacco

According to the method of Example 3, a dried solid containing CBT from which alkaloids had been removed was produced. The concentration of CBT in the dried solid was about 99%. About 100 ppm by weight of the dried solid was added to tobacco base sheet shreds. The obtained flavored sheet shreds were dried to obtain flavored sheet shreds that can be subjected to smoking evaluation. A non-combustion heating type flavor inhalation article was prepared using the flavored sheet shreds. The article was heated from the outside and smoking evaluation was performed. Smoking evaluation was performed by five well-trained panelists of an average age of 48 years. In the evaluation method, the intensity of the tobacco aroma was used as an index, and the evaluation was performed based on the following scores of: 1 point (no change) as no aroma; 2 points (strong); and 3 points (very strong). The test of the difference between the average values in the score was performed by two-sided test. As a result, it was confirmed that a yellowish flavor peculiar to tobacco was imparted.

### [Example 7] Confirmation of effect with cigarette

About 100 ppm by weight of the dried solid obtained in Example 3 was added to shredded tobacco. The cased shreds were dried to obtain cased shreds that can be subjected to smoking evaluation. A cigarette using the cased shreds was prepared by an ordinary method, and smoking evaluation was performed in the same manner as in Example 6. The results demonstrated that the cased shreds exhibited better original tobacco aroma.

### REFERENCE SIGNS LIST

- 10: Heating apparatus
- 11: Body
- 12: Heater
- 13: Metal tube
- 14: Battery unit
- 15: Control unit
- 16: Recess
- 17: Vent hole

- 20: Non-combustion heating type flavor inhalation article
- 20A: Tobacco rod part
- 20B: Cooling part
- 20C: Filter part

- 21: Tobacco filler
- 22: Cigarette paper
- 23: Paper tube
- 24: Perforation
- 25: First segment
- 25a: First filling layer
- 25b: Inner plug wrapper
- 26: Second segment
- 26a: Second filling layer
- 26b: Inner plug wrapper
- 27: Outer plug wrapper
- 28: Lining paper

- 30: Non-combustion non-heating type flavor inhalation article
- 30D: Power supply unit
- 30E: Cartridge
- 30F: Tobacco capsule
- u: Non-inhalation end
- d: Inhalation end
- 110: Battery
- 200: Aerosol source
- 210: Reservoir
- 220: Atomizer
- 230: Flow path forming body
- 240: Outer frame 240
- 250: End cap
- 200X: First flow path
- 300: Flavor source
- 310: Container
- 320: Mesh body
- 330: Nonwoven fabric
- 340: Cap

## Claims

1. A method for producing a tobacco extract containing cembratrienediol, the method comprising:
a process 1 that includes preparing a discharged solid obtained by an expansion treatment of a tobacco raw material;
a process 2 that includes subjecting the discharged solid to solid-liquid extraction with an aprotic solvent; and
a process 3 that includes recovering an organic phase from the process 2.

2. The production method according to claim 1, wherein the process 2 further includes subjecting an aprotic solvent phase obtained by the solid-liquid extraction to extraction with water or an acid aqueous solution.

3. The production method according to claim 1 or 2, wherein the aprotic solvent is water-insoluble.

4. The production method according to claim 3, wherein the water-insoluble organic solvent is a hydrocarbon having 5 or 6 carbon atoms.

5. The production method according to any one of claims 1 to 4, further comprising:
a process P1 that includes subjecting an organic phase obtained in the process 2 or a tobacco extract obtained by removing the solvent from the organic phase to normal phase chromatography using a mixed solvent of n-hexane and ethyl acetate as a mobile phase, to obtain a fraction of n-hexane and ethyl acetate (50 : 50) and a fraction of n-hexane and ethyl acetate (40 : 60).

6. The production method according to any one of claims 1 to 4, further comprising:
a process P2 that includes adding methanol or ethanol to an organic phase obtained in the process 2 or a tobacco extract obtained by removing the solvent from the organic phase to produce a precipitate and obtaining a solution from which the precipitate has been removed.

7. The production method according to claim 5 or 6, further comprising:
a process P3 that includes subjecting the fraction obtained in the process P1 or P2 to reverse phase chromatography using water and methanol as a mobile phase.

8. The production method according to any one of claims 2 to 7, wherein the acid aqueous solution is an aqueous solution of sulfuric acid, citric acid, or oxalic acid.

9. The production method according to any one of claims 2 to 8, wherein a pH of the acidic aqueous solution is 3 or less.

10. A tobacco extract comprising cembratrienediol produced by the method according to any one of claims 1 to 9.

11. The tobacco extract according to claim 10, wherein the tobacco extract contains 90 wt% or more of the cembratrienediol.

12. A tobacco flavoring agent comprising the tobacco extract according to claim 10 or 11.

13. A tobacco material comprising the tobacco flavoring agent according to claim 12.

14. The tobacco material according to claim 13, wherein the tobacco material is a tobacco sheet or a shredded tobacco.

15. A tobacco rod part comprising the tobacco material according to claim 13 or 14.

16. A tobacco flavor inhalation article comprising the tobacco rod part according to claim 15.

17. A non-combustion heating type tobacco flavor inhalation article or a non-combustion non-heating type tobacco flavor inhalation article comprising the tobacco rod part according to claim 15.

18. A smokeless tobacco comprising the tobacco material according to claim 13 or 14.
